# EUROPÄISCHE PATENTSCHRIFT

(11) **EP 3 666 380 B1**
(45) Veröffentlichungstag und Bekanntmachung des Hinweises auf die Patenterteilung: **01.05.2024**
(21) Anmeldenummer: 19214466.5
(22) Anmeldetag: 09.12.2019
(51) Int. Cl.: B01L 3/02, A61M 5/31

(54) **VORRICHTUNG UND VERFAHREN ZUM DOSIEREN VON CHEMIKALIEN**
DEVICE AND METHOD FOR METERING OF CHEMICALS
DISPOSITIF ET PROCÉDÉ DE DOSAGE DE PRODUITS CHIMIQUES

(30) Priorität: 10.12.2018 DE 102018131603
(43) Veröffentlichungstag der Anmeldung: 17.06.2020
(73) Patentinhaber: HiTec Zang GmbH, 52134 Herzogenrath (DE)
(72) Erfinder: TRUTNAU, Mirko, 52134 Herzogenrath (DE); FUNKE, Tobias, 52134 Herzogenrath (DE); MASSBERG, Rolf, 52134 Herzogenrath (DE); PAQUET, Markus, 52134 Herzogenrath (DE); KO, Jae, 52134 Herzogenrath (DE); NÜCHTER, Marc, 52134 Herzogenrath (DE); ZANG, Christoph, 52134 Herzogenrath (DE)
(74) Vertreter: Fitzner, Uwe

(56) Entgegenhaltungen:
- WO-A1-99/21599
- US-A- 679 198
- US-A- 2 813 528
- US-A1- 2014 249 410

## Beschreibung

Die vorliegende Erfindung betrifft eine Vorrichtung zur Dosierung von Flüssigkeiten und Chemikalien mittels eines Spritzendosierers.

In der Biologie und Chemie werden vorzugsweise Glas (Borosilikatglas) und Fluor-Kunststoffe wie Polytetrafluorethylen (PTFE) oder auch Thermoplasten (z.B. PEEK) als medienführende Teile verwendet. Bei der Berührung mit Medien ist für diese Verbindungen eine hohe Verträglichkeit zu verzeichnen.

Bei dieser Vorrichtung wird der Kunststoff als Dichtung verwendet. Für die Verbindung zwischen Glas und Kunststoff werden in der Praxis Klebverbindungen eingesetzt. Diese sind aber nicht gegen alle Chemikalien beständig. D.h. es kann zu Ablösungserscheinungen infolge des Einsatzes von Chemikalien oder auch von Druck kommen, so dass die Dosierspritze undicht wird.

Die Verbindung von Glas und Metall ist dem Fachmann bekannt. So werden beispielsweise in der DE 10 2008 042 80 A1 entsprechend EP 2 165 986 B1 spezielle Glaszusammensetzungen vorgestellt, die für solche Verbindungen geeignet sind.

Als weiterer Stand der Technik könnten beispielsweise DE 10 2013 210 998 A1, DE 23 47 500 A, DE 20 2007 013 096 U1, US 679 198 A, US 2 813 528 A, WO 99/21599 A1 oder auch DE 1 129 717 A genannt werden.

Problematisch nach dem Stand der Technik ist es, dass Klebverbindungen sich lösen. Dies gilt insbesondere in dem Fall, dass eine Dosierung unter hohem Druck durchgeführt werden soll. Abgesehen davon können Unregelmäßigkeiten, nicht genaues Aufsetzen usw., zu einer verringerten Belastbarkeit führen.

Aufgabe der vorliegenden Erfindung ist es nunmehr, diese Nachteile zu vermeiden. Insbesondere soll die Verbindung der Bauteile selbst gegen aggressive Chemikalien beständig sein und auch hohe Drucke und hohe Temperaturen ohne eine Lösung des Verbindungsmechanismus Bestand haben.

Diese und weitere Aufgaben, die sich dem Fachmann bei Betrachtung der folgenden Beschreibung erschließen, werden durch die Gegenstände der unabhängigen Ansprüche gelöst. Vorteilhafte Weiterbildungen ergeben sich aus den Unteransprüchen und der folgenden Beschreibung.

Gegenstand der vorliegenden Erfindung ist mithin eine Vorrichtung zur Dosierung von Fluiden mittels einer Dosierspritze, aufweisend einen durch ein Glasrohr geführten Spritzenkolben, einen Spritzenkopf der kraftschlüssig und klebstofffrei mittels einer Pressverbindung am Glasrohr angeordnet ist, bei welcher der Spritzenkolben mit einer Umhüllung versehen ist, und an den Enden der Umhüllung Deckel angeordnet sind. Diese Vorrichtung weist an den Deckeln und den Enden der Umhüllung miteinander kompatible Schraubgewinde auf. Ferner ist der Spritzenkopf von innen durch den Spritzendeckel nach außen geführt.

Gegenstand der vorliegenden Offenbarung ist auch eine Vorrichtung zur Dosierung von Flüssigkeiten und Chemikalien mittels einer Dosierspritze gelöst, aufweisend einen durch ein Glasrohr geführten Spritzenkolben mit einem Spritzenkopf, der kraftschlüssig und klebstofffrei mittels einer Pressverbindung am Glasrohr angeordnet ist.

Das Glasrohr wird erfindungsgemäß von einer Umhüllung ummantelt. Diese kann aus beliebigen Materialien, vorzugsweise aus Metall gefertigt sein, oder Metall enthalten. Als Metalle für die Umhüllung kommen beispielsweise Edelstahl, Aluminium oder Aluminiumlegierungen in Betracht. Ebenso kommen Kunststoffe für die Umhüllung in Betracht.

In einigen Ausgestaltungen der vorliegenden Erfindung wird der Kunststoff für die Umhüllung ausgewählt aus der Gruppe bestehend aus Polycarbonat, Polypropylen, Cycloolefin-Copolymeren, Polystyrol, Polyaryletherketonen und Gemischen sowie Verbunden davon eingesetzt.

In einer Ausgestaltung der vorliegenden Erfindung werden Polyetheretherketone (PEEK) zur Herstellung der Umhüllung eingesetzt. Die Umhüllung kann in einigen Ausgestaltungen aus PEEK bestehen.

Gleichsam ist es möglich, dass die Umhüllung aus Kombinationen dieser Stoffe besteht oder diese enthält. In einer Ausgestaltung der vorliegenden Erfindung wird eine Umhüllung aus Metall gefertigt, die über Ihre Länge hinweg eine Aussparung aufweist, in welche dann Kunststoff eingefügt ist; dies entspricht einer Ausgestaltung mit seitlichem Fenster. In einer weiteren Ausgestaltung der vorliegenden Erfindung wird eine Umhüllung aus Kunststoff gefertigt und Metall lediglich zur Verstärkung in die Umhüllung eingearbeitet bzw. außen aufgebracht, z.B. in Form von Metallringen. Im Rahmen der vorliegenden Erfindung einsetzbare Polyetheretherketone sind hochtemperaturbeständige thermoplastische Kunststoffe, welche zur Stoffgruppe der Polyaryletherketone gehören. Deren Schmelztemperatur bzw. Schmelzbereich liegt in einigen Fällen bei etwa 335°C.

Die Umhüllung kann durchsichtig sein, ist aber vorzugsweise nicht durchsichtig. In diesem Falle ist es daher bevorzugt, an den Seiten Fenster anzuordnen, um das Ablesen von Werten, z.B. einer angeordneten Skala, zu ermöglichen.

An den Enden der Umhüllung sind Deckel angeordnet. Diese weisen mit den Enden der Umhüllung kompatible Schraubgewinde auf, so dass eine Verschraubung ermöglicht wird. Vorzugsweise sind die Deckel so beschaffen, dass sie zerstörungsfrei und wieder lösbar angeordnet sind. Im Ergebnis weist die Vorrichtung einen Spritzenkolbendeckel auf, welcher mit der Umhüllung verschraubt ist. Ferner weist sie einen Spritzendeckel auf, welcher ebenfalls mit der Umhüllung verschraubt wird. Hierbei wird der Spritzenkopf von innen durch den Spritzendeckel nach außen geführt.

Im Inneren der Umhüllung befindet sich ein Glasrohr. Der Spritzenkolben wird entsprechend dem bisherigen Stand der Technik durch das Glasrohr geführt. Zur Vermeidung von direkten Kontaktflächen von Metall auf Glas kann eine Kunststoffscheibe zwischen dem Kolbendeckel und dem Glasrohr platziert werden.

Im Weiteren kann eine Spritzendichtung in das Glasrohr eingeführt werden. Die Dichtung kann aus dem Fachmann bekannten Materialien bestehen. Insbesondere kommen Kunststoffe in Betracht. Hierzu zählen Elastomere, Duroplaste und Thermoplaste. In Betracht kommen auch weiche Materialien, z.B. weiche Metalle oder durch die Geometrie elastisch gestaltete Metalle. Ebenso können auch Fluorpolymere verwendet werden. Hierzu zählen Polytetrafluorethylen (PTFE)oder Perfluoralkoxy-Polymere (PFA). In Betracht kommen auch Fluor-Verbindungen wie Fluor-Kautschuk oder Fluor-Elastomere. Hierzu zählen z.B. Fluor-Kautschuke (FKM) oder Perfluorkautschuk (FFKM). Weiterhin kommen andere Kunststoffe wie Ultrahochmolekulares Polyethylen (UHMWPE) in Betracht. Weiterhin kommen auch verschiedene Metalle, vorzugsweise Gold oder Edelstahl in Betracht. Diese Aufzählung ist nicht abschließend. Auch kommen beliebige Kombinationen der betreffenden Materialien in Betracht.

Die Dichtfläche des Spritzenkolbens mit dem Spritzenkopf sorgt durch das Anpressen für eine Dichtung. Der Glaskörper wird zentriert und es ist somit gewährleistet, dass die Öffnung des Spritzenkopfes genau über die Öffnung der Spritzendichtung liegt.

Erfindungsgemäß wird das Anpressen durch eine Verschraubung erreicht.

In Varianten der vorliegenden Offenbarung sind auch alle anderen dem Fachmann bekannten Pressverfahren anwendbar.

Die Verschraubung bietet unter anderem den Vorteil, dass die Verbindung und auch das Lösen sehr leicht bewerkstelligt werden können. Durch geeignete mechanische Methoden kann ohne große Kraftanstrengung auch ein festes Anziehen erreicht werden. Die mechanische Belastbarkeit der Spritze wird stark erhöht. Hohe Kräfte, welche durch das Zusammenpressen des Fluids durch den Kolben in dem Glasrohr entstehen können, werden hiermit kompensiert. Bei den erfindungsgemäß Schraubverbindungen ist es jederzeit möglich, den Anpressdruck neu zu justieren oder nach zu justieren. Ebenso ermöglicht die Verschraubung ein einfaches Lösen der Verbindung, wodurch eine Wartung der Vorrichtung einfach ist und wodurch auch jederzeit der innere Teil der Vorrichtung, also im Wesentlichen das Glasrohr, der Spritzenkolben oder die Umhüllung getauscht werden können. Dies bedeutet eine sehr leichte und flexible Wartung; ebenso ist es durch die erfindungsgemäß Ausgestaltung möglich, eine Art Spritzenkit zur Verfügung zu stellen, bei dem für die verschiedenen Komponenten der Vorrichtung jeweils unterschiedliche Ausgestaltungen vorgehalten und bei Bedarf die Vorrichtung entsprechend zusammengesetzt wird.

Damit weist das erfindungsgemäße System nicht die Nachteile des Standes der Technik auf.

Eine weitere Ausgestaltung der vorliegenden Erfindung ist demgemäß ein Spritzenkit, welches als Bestandteile mindestens ein Glasrohr, mindestens einen Spritzenkolben, mindestens einen Spritzenkopf, mindestens eine Umhüllung mit Schraubgewinden an beiden Enden und mindestens zwei Deckel mit Schraubgewinden, die mit den Schraubgewinden der mindestens einen Umhüllung kompatibel sind, umfasst.

Bei diesem Kit sind die einzelnen Elemente einerseits in ihren Dimensionen aufeinander abgestimmt und liegen andererseits jeweils in verschiedenen Ausgestaltungen, insbesondere im Hinblick auf Materialien bzw. Materialkombinationen, vor. Dadurch wird eine hohe Flexibilität erreicht. Gegebenenfalls kann dieses Kit noch Nadeln zum Anbringen an dem Spritzenkopf umfassen. Ferner ist dieses Kit zur Herstellung von Vorrichtungen gemäß einem der Ansprüche 1 bis 8.

Die Vorrichtung zur Dosierung kann in hohen Druckbereichen eingesetzt werden (nachfolgende Angaben als absolute Drucke). Hier lassen sich ohne Weiteres Bereiche von 0 - 300 bar, oder von 100 bis 200 bar als Einsatzgebiete nennen und realisieren. Erfindungsgemäß kann vorzugsweise mit einem Druck bis zu 270 bar ohne weiteres gearbeitet werden. Für die Temperaturbereiche gilt das Entsprechende. Die Spritzen können in bevorzugten Ausführungsformen von -20°C bis + 230°C zum Einsatz kommen.

Die erfindungsgemäßen Vorrichtungen sind für Flüssigkeiten, Emulsionen, Suspensionen geeignet. Einziger begrenzender Faktor ist, dass das verwendete Glas und die verwendeten Kunststoffe inert gegen die eingesetzten chemischen Verbindungen sind.

Erfindungswesentlich ist im Weiteren, dass kein Metall in Kontakt mit den chemischen Medien, welche mittels der erfindungsgemäßen Vorrichtung dosiert werden, in Berührung kommt. Weiterer Vorteil gegenüber dem Stand der Technik ist, dass auch flüssige organische Verbindungen mit der erfindungsgemäßen Vorrichtung dosiert werden können. Auch hier ist der erfindungsgemäße Vorteil darin zu sehen, dass das spezielle Dichtungssystem nicht angegriffen wird. Denn insbesondere waren Klebestoffverbindungen die Schwachstelle bei der Verwendung von aggressiven Fluiden.

In manchen Varianten der vorliegenden Erfindung umfasst die Vorrichtung zusätzlich eine Nadel, die an dem Spritzenkopf angebracht ist. Dies kann bevorzugt eine Hohlnadel sein, insbesondere wie sie aus dem Stand der Technik bekannt sind. Gegebenenfalls können die Nadel und der Spritzenkopf aufeinander abgestimmte Verbindungselemente aufweisen.

In Varianten der vorliegenden Erfindung erstreckt sich der Injektionskolben bzw. Spritzenkolben nur bis zum unteren Ende des Glaskolbens, aber nicht bis in den Spritzenkopf hinein.

In weiteren Varianten der vorliegenden Erfindung erstreckt sich der Injektionskolben bzw. Spritzenkolben nur bis zum unteren Ende des Spritzenkopfs, aber nicht bis in eine aufgesetzte Nadel hinein.

In bevorzugten Varianten der vorliegenden Erfindung wird gänzlich auf den Einsatz von Federn zur Erzeugung von Klemmkräften verzichtet, d.h. die Vorrichtung umfasst keine Federn.

In bevorzugten Varianten der vorliegenden Erfindung ist das verwendete Glasrohr dickwandig. Dabei bedeutet "dickwandig" ein Verhältnis von Außendurchmesser zu Innendurchmesser von größer oder gleich 1,2 (D/d >=1,2). Dies ermöglicht, dass das Glasrohr höhere Kräfte aushält, ohne zu versagen. Dies ist vorteilhaft, weil dadurch in noch höherem Maße für das Abdichten bei höheren Betriebsdrucken erforderliche, hohe Kräfte auf den Glaszylinder aufgebracht werden können. In Kombination mit einer Umhüllung kann diese Druckwiderstandsfähigkeit nochmals gesteigert werden.

Bei Einsatz einer Schraubverbindung ist von Vorteil, dass die Dichtung auch bei längerer Verwendung mit Hilfe des Spritzen- und Kolbendeckels einfach nachgezogen werden kann. Dies war bei Klebverbindungen nicht möglich. Durch den modularen Aufbau des Systems ist es auch möglich, die verschlissenen Teile wieder auszutauschen. Ebenso ist ein solcher Austausch möglich, wenn ein Materialwechsel vonnöten ist. So kann der Spritzenkopf eines bestimmten Materials gegen einen anderen mit einem anderen Material ausgetauscht werden, wenn dies aufgrund des Wechsels des zu dosierenden Fluids vonnöten ist. Ebenso wird das Reinigen der Bauteile durch den modularen Aufbau erleichtert. Auch können erfindungsgemäß Systeme mit unterschiedlichem Volumen von ein und derselben Umhüllung umgeben werden, so dass es nicht vonnöten ist, für jede Spritze eine separate Umhüllung zu verwenden.

Bei der vorliegenden Erfindung ist die innere Spritzendichtung 8 in vorteilhaften Ausgestaltungen zumindest zum Teil in das Glasrohr eingeführt, wodurch eine besonders vorteilhafte Abdichtung erreicht wird. Bei hohem Innendruck erzielt dies eine bessere Abdichtung als nur aufgelegte Dichtungen z.B. in Form von Scheiben. Beispielsweise kann die Dichtung in Form eines Hohlstopfens ausgeführt sein.

Zu den besonderen Vorteilen gehört auch, dass eine Vorkehrung gegen das Bersten des Glaskörpers oder der Verbindungen zwischen Glaskörper und Metall nicht erforderlich ist, falls das System bei einem hohen Druck, beispielsweise von 30 bar oder mehr zu platzen droht. Dieser Nachteil tritt nämlich bei Verklebungen auf. Weiterhin ist ein Vorteil der vorliegenden Erfindung, dass die das Glasrohr umgebende Hülle als Splitterschutz dient, für den Fall, dass das Glasrohr brechen sollte. Dieser Schutz wird durch die sichere Verbindung durch die geschraubten Deckel im Vergleich zu Ausgestaltungen ohne Deckel oder mit "nur" gesteckten Deckeln noch verbessert.

Vorteilhaft bei der vorliegenden Erfindung gegenüber Vorrichtungen, bei denen z.B. die Deckel nur aufgeklemmt sind, ist, dass solche Klemmlösungen im Gegensatz zur vorliegenden Erfindung nicht kontrollier voneinander lösbar sind, d.h. sie sind oft so fest verbunden, dass sie nicht zerstörungsfrei lösbar sind oder aber sie sind zu leicht lösbar, können also keine Fixierung oder Sicherheit gewährleisten.

Die vorliegende Offenbarung betrifft auch folgende Ausgestaltung:
Es wird eine Vorrichtung zur Dosierung von Fluiden mittels einer Dosierspritze offenbart. Dies Vorrichtung weist einen durch ein Glasrohr geführten Spritzenkolben mit einem Spritzenkopf auf. Dieser kann kraftschlüssig und klebstofffrei mittels einer Pressverbindung am Glasrohr angeordnet sein.

Gleichsam kann der Spritzenkopf in dieser Ausführungsform der vorliegenden Offenbarung zerstörungsfrei wieder lösbar sein. Zudem kann der Spritzenkolben mit einer Umhüllung versehen sein.

Die in diesem Zusammenhang einsetzbare Umhüllung kann aus Metall gefertigt sein oder Metall enthalten, wobei sie in bevorzugten Varianten aus Edelstahl, Aluminium oder Aluminium-Legierungen oder Kunststoffen oder beliebigen Kombinationen dieser Stoffe bestehen oder diese enthalten kann.

Es ist dabei möglich, die Umhüllung so zu gestalten, dass sie mindestens ein Sichtfenster aufweist.

Auch bei dieser Ausgestaltung der vorliegenden Offenbarung ist es möglich, an den Enden der Umhüllung Deckel anzuordnen, die dann mit den Enden der Umhüllung kompatible Schraubgewinde aufweisen können.

Selbstverständlich ist es auch hier möglich zwischen dem Kolbendeckel und dem Ende des Glasrohrs des Spritzenkolbens eine Dichtungsscheibe anzuordnen.

Weiterhin kann in dieser Ausgestaltung der vorliegenden Offenbarung die Dichtung aus Kunststoffen, vorzugsweise Elastomeren, Duroplasten, Thermoplasten oder weichen Metallen oder elastisch gestalteten Metallen bestehen, oder diese Stoffe oder ein Gemisch dieser Stoffe enthalten. Bevorzugt ist es in dieser Ausgestaltung, wenn die Dichtung Fluor-Polymere aufweist.

Die Vorrichtung gemäß dieser Ausgestaltung der vorliegenden Offenbarung kann zur Dosierung von Fluiden verwendet werden.

Im Folgenden wird die Erfindung anhand der Figuren näher beschrieben. Die Figuren sind dabei nicht maßstabsgetreu und nicht wesentliche Elemente werden nicht unbedingt dargestellt. Die Figuren sind darüber hinaus nur zur Illustration gedacht und schränken die vorliegende Erfindung nicht auf diese speziellen Ausführungsformen ein.

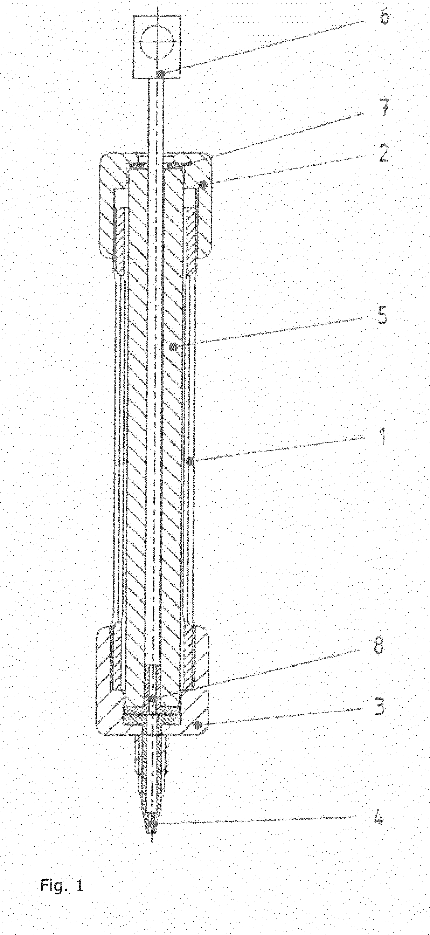

Figur 1 zeigt eine erfindungsgemäße Vorrichtung in Schnittdarstellung. Mit der Ziffer 1 ist eine Hülle bezeichnet, welche vorzugsweise aus Metall besteht oder dieses enthält. Nicht dargestellt sind die Fenster, welche beispielsweise dem Ablesen einer Scala dienen können, sofern die Umhüllung undurchsichtig ist. An die Umhüllung 1 wird der Spritzkolbendeckel 2 geschraubt. Mit der Umhüllung 1 wird im Weiteren der Spritzendeckel 3 verschraubt. Durch den Spritzendeckel 3 wird der Spritzenkopf 4 geführt. Im Inneren der Umhüllung 1 ist das Glasrohr 5 angeordnet. Der Spritzenkolben 6, der hier mit einem viereckigen Kopf dargestellt ist (dieser dient zur besseren Bedienbarkeit), wird durch das Glasrohr 5 geführt. Zwischen dem Spritzkolbendeckel 2 und dem Glasrohr 5 ist eine Kunststoffscheibe 7 angeordnet. Die Spritzendichtung 8 ist teilweise in das Innere des Glasrohrs 5 eingeführt. Mit Hilfe der durch die beiden Deckel 2 und 3 in Verbindung mit der Hülle 1 erzeugten Andruckkraft wird sie an die Dichtfläche des Spritzenkopfes 4 gepresst.

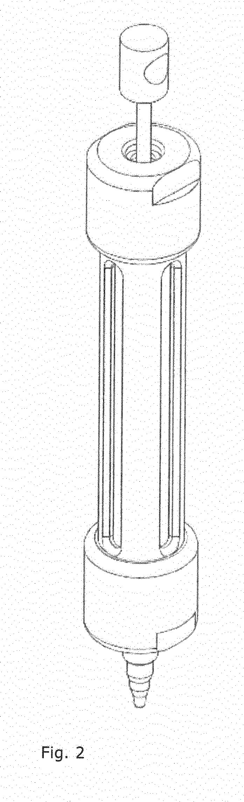

Figur 2 ist eine schräge Draufsicht auf eine Vorrichtung gemäß vorliegender Erfindung. In dieser Illustration sind in der Umhüllung zwei längliche Vertiefungen dargestellt, die als (Sicht-)Fenster dienen können und wahlweise mit einem insbesondere durchsichtigen Material ausgefüllt sein können, was in dieser Figur nur angedeutet ist.

### Bezugszeichenliste:

- 1 -: Umhüllung
- 2 -: Kolbendeckel
- 3 -: Spritzendeckel
- 4 -: Spritzenkopf
- 5 -: Glasrohr
- 6 -: Spritzenkolben
- 7 -: Dichtscheibe
- 8 -: Spritzendichtung

## Patentansprüche

1. Vorrichtung zur Dosierung von Fluiden mittels einer Dosierspritze, aufweisend einen durch ein Glasrohr (5) geführten Spritzenkolben (6), einen Spritzenkopf (4) der kraftschlüssig und klebstofffrei mittels einer Pressverbindung am Glasrohr (5) angeordnet ist,
wobei das Glasrohr (5) mit einer Umhüllung (1) versehen ist, und
wobei an den Enden der Umhüllung (1) Deckel (2,3) angeordnet sind, **dadurch gekennzeichnet, dass** die Deckel (2,3) und die Enden der Umhüllung (1,2) miteinander kompatible Schraubgewinde aufweisen, **dadurch gekennzeichnet dass** der Spritzenkopf (4) von innen durch den Spritzendeckel (3) nach außen geführt wird.

2. Vorrichtung nach Anspruch 1, **dadurch gekennzeichnet, dass** die Umhüllung (1) aus Metall gefertigt ist oder Metall enthält.

3. Vorrichtung nach Anspruch 2, **dadurch gekennzeichnet, dass** die Umhüllung aus Edelstahl, Aluminium oder Aluminium-Legierungen oder Kunststoffen oder beliebigen Kombinationen dieser Stoffe besteht oder diese enthält.

4. Vorrichtung nach Anspruch 1 bis 3, **dadurch gekennzeichnet, dass** die Umhüllung (1) mindestens ein Sichtfenster aufweist.

5. Vorrichtung nach einem der vorhergehenden Ansprüche, **dadurch gekennzeichnet, dass** zwischen dem Kolbendeckel (2) und dem Ende des Glasrohrs (5) des Spritzenkolbens (6) eine Dichtungsscheibe (7) angeordnet ist.

6. Vorrichtung nach Anspruch 5, **dadurch gekennzeichnet, dass** die Dichtung (7) aus Kunststoffen, vorzugsweise Elastomeren, Duroplasten, Thermoplasten oder weichen Metallen oder elastisch gestalteten Metallen besteht, oder diese Stoffe oder ein Gemisch dieser Stoffe enthält.

7. Vorrichtung nach Anspruch 6, **dadurch gekennzeichnet, dass** die Dichtung (7) Fluor-Polymere aufweist.

8. Vorrichtung nach einem der vorhergehenden Ansprüche, **dadurch gekennzeichnet, dass** die Vorrichtung eine Nadel zur Anbringung an dem Spritzenkopf umfasst.

9. Verwendung der Vorrichtungen nach einem der vorhergehenden Ansprüche zur Dosierung von Fluiden.

10. Spritzenkit umfassend die folgenden Bestandteile,
a) mindestens ein Glasrohr,
b) mindestens einen Spritzenkolben,
c) mindestens einen Spritzenkopf,
d) mindestens eine Umhüllung mit Schraubgewinden an beiden Enden,
e) mindestens zwei Deckel mit Schraubgewinden, die mit den Schraubgewinden der mindestens einen Umhüllung kompatibel sind,
f) optional mindestens eine Nadel zur Anbringung an dem mindestens einen Spritzenkopf,
wobei die Elemente a) bis f) in ihren Dimensionen aufeinander abgestimmt sind und die Elemente b) bis f) jeweils in verschiedenen Ausgestaltungen, insbesondere im Hinblick auf Materialien bzw. Materialkombinationen, vorliegen,
zur Herstellung von Vorrichtungen gemäß einem der Ansprüche 1 bis 8.

## Claims

1. Device for metering of fluids by means of a metering syringe, having a syringe plunger (6) guided through a glass tube (5), a syringe head (4) which is arranged on the glass tube (5) in a force-fitting and adhesive-free manner by means of a press connection,
wherein the glass tube (5) is provided with a sheath (1), and
wherein lids (2, 3) are arranged at the ends of the sheath (1), **characterised in that** the lids (2, 3) and the ends of the sheath (1, 2) have screw threads which are compatible with one another,
**characterised in that** the syringe head (4) is guided from the inside through the syringe lid (3) to the outside.

2. Device according to claim 1, **characterised in that** the sheath (1) is made of metal or contains metal.

3. Device according to claim 2, **characterised in that** the sheath consists of or contains stainless steel, aluminium or aluminium alloys or plastics or any combination of these materials.

4. Device according to claim 1 to 3, **characterised in that** the sheath (1) has at least one viewing window.

5. Device according to one of the preceding claims, **characterised in that** a sealing disc (7) is arranged between the plunger lid (2) and the end of the glass tube (5) of the syringe plunger (6).

6. Device according to claim 5, **characterised in that** the seal (7) consists of plastics, preferably elastomers, thermosets, thermoplastics or soft metals or elastically shaped metals, or contains these substances or a mixture of these substances.

7. Device according to claim 6, **characterised in that** the seal (7) comprises fluoropolymers.

8. Device according to one of the preceding claims, **characterised in that** the device comprises a needle for attachment to the syringe head.

9. Use of the devices according to one of the preceding claims for metering of fluids.

10. Syringe kit comprising the following components,
a) at least one glass tube,
b) at least one syringe plunger,
c) at least one syringe head,
d) at least one sheath with screw threads at both ends,
e) at least two lids with screw threads that are compatible with the screw threads of the at least one sheath,
f) optionally at least one needle for attachment to the at least one syringe head,
wherein the dimensions of the elements a) to f) are matched to one another and the elements b) to f) are each present in different configurations, in particular with regard to materials or material combinations,
for the manufacture of devices according to one of claims 1 to 8.

## Revendications

1. Dispositif de dosage de fluides au moyen d'une seringue de dosage, comprenant un piston de seringue (6) guidé à travers un tube en verre (5), une tête de seringue (4) agencée sur le tube en verre (5) par adhérence et sans colle au moyen d'un assemblage serré,
le tube en verre (5) étant pourvu d'une enveloppe (1), et
des couvercles (2, 3) étant disposés aux extrémités de l'enveloppe (1),
**caractérisé en ce que** les couvercles (2, 3) et les extrémités de l'enveloppe (1, 2) présentent des filets de vis compatibles entre eux,
**caractérisé en ce que** la tête de seringue (4) est guidée de l'intérieur vers l'extérieur à travers le couvercle de seringue (3).

2. Dispositif selon la revendication 1,
**caractérisé en ce que** l'enveloppe (1) est fabriquée en métal ou contient du métal.

3. Dispositif selon la revendication 2,
**caractérisé en ce que** l'enveloppe est constituée ou contient de l'acier inoxydable, de l'aluminium ou des alliages d'aluminium ou des matières plastiques ou toute combinaison de ces matériaux.

4. Dispositif selon les revendications 1 à 3,
**caractérisé en ce que** l'enveloppe (1) présente au moins une fenêtre d'observation.

5. Dispositif selon l'une des revendications précédentes,
**caractérisé en ce qu'**une rondelle d'étanchéité (7) est disposée entre le couvercle de piston (2) et l'extrémité du tube de verre (5) du piston de seringue (6).

6. Dispositif selon la revendication 5,
**caractérisé en ce que** le joint d'étanchéité (7) est constitué de matières plastiques, de préférence d'élastomères, de thermodurcissables, de thermoplastiques, ou de métaux doux ou de métaux de conception élastique, ou contient ces matériaux ou un mélange de ces matériaux.

7. Dispositif selon la revendication 6,
**caractérisé en ce que** le joint d'étanchéité (7) comprend des polymères fluorés.

8. Dispositif selon l'une des revendications précédentes,
**caractérisé en ce que** le dispositif comprend une aiguille destinée à être fixée à la tête de la seringue.

9. Utilisation des dispositifs selon l'une des revendications précédentes pour le dosage de fluides.

10. Kit de seringue comprenant les composants suivants :
a) au moins un tube en verre,
b) au moins un piston de seringue,
c) au moins une tête de seringue,
d) au moins une enveloppe présentant des filets de vis aux deux extrémités,
e) au moins deux couvercles présentant des filets de vis compatibles avec les filets de vis de ladite au moins une enveloppe,
f) optionnellement, au moins une aiguille destinée à être fixée à ladite au moins une tête de seringue,
les éléments a) à f) étant adaptés les uns aux autres quant à leurs dimensions, et les éléments b) à f) se présentant chacun dans différentes conceptions, en particulier en ce qui concerne les matériaux ou les combinaisons de matériaux,
pour la fabrication de dispositifs selon l'une des revendications 1 à 8.
